# EUROPEAN PATENT APPLICATION

(11) **EP 3 903 687 A1**
(43) Date of publication of application: **03.11.2021**
(21) Application number: 19906284.5
(22) Date of filing: 25.07.2019
(51) Int. Cl.: A61B 17/00

(54) **LOCKING DEVICE HAVING LOCKING FEEDBACK FUNCTION, AND CARDIAC VALVE REPAIR SYSTEM**

(30) Priority: 26.12.2018 CN 201811599592; 26.12.2018 CN 201822205997 U
(71) Applicant: Hangzhou Valgen Medtech Co., Ltd., Hangzhou, Zhejiang 310052 (CN)
(72) Inventor: ZHANG, Tingchao, Hangzhou, Zhejiang 310052 (CN); ZHENG, Xianzhang, Hangzhou, Zhejiang 310052 (CN); ZHANG, Weiwei, Hangzhou, Zhejiang 310052 (CN)
(74) Representative: Hamer, Christopher K.
(86) International application number: PCT/CN2019/097747
(87) International publication number: WO 2020/134060

(57) **Abstract**

A locking device with a locking feedback function is provided. The locking device includes a locking assembly, a handle, and a locking feedback element. The locking assembly is configured to lock a target object. The locking assembly is coupled with the handle. The handle comprises a movable portion and a fixed portion. The movable portion is configured to move relative to the fixed portion to drive the locking assembly to lock the target object. The locking feedback element is movably disposed in the fixed portion, and is configured to cooperate with the movable portion of the handle to feed back whether the target object is locked. The disclosure further provides a heart valve repair system. When the locking device with the locking feedback function in the disclosure is used to lock the target object, whether the target object is locked by the locking assembly may be judged through the locking feedback element to ensure a locking degree of the target object, so that a locking effect is ensured, and the success rate of the operation is increased.

## Description

### TECHNICAL FIELD

The disclosure relates to the field of medical equipment, and particularly to a locking device with a locking feedback function and a heart valve repair system.

### BACKGROUND

During surgery, an operation step of knotting and fixing sutures is often required. A traditional surgical operation is performed under direct vision, and the sutures may usually be knotted manually by a doctor. However, with the development of technology, various minimally invasive surgeries and interventional surgeries such as an endoscopic surgery and a transcatheter interventional surgery are increasingly becoming common. For these surgeries, patient's body only needs to be incised to form a relatively small incision that acts as an operating window, and through the operating window instruments such as endoscopes or interventional catheters are pushed into the patient's body until the instruments reach a predetermined position to perform treatment. For these surgeries, if the sutures need to be knotted or fixed, the operator usually needs to perform, through the relatively small operating window, a remote operation outside the patient's body to knot the sutures disposed in the patient's body. In the related art, sutures are usually knotted or fixed at long distances by surgical robots, knot pushers, transcatheter suture lock implanting systems, or other methods.

Common implantable sutures are usually made from Polyethylene Terephthalate (PET) or Polytetrafluoroethylene (PTFE). A PTFE suture is usually a single-stranded line, and PTFE is a polymer material, so when the PTFE suture is excessively locked by a metal piece such as a lock and a lock pin, a pressure point is easily damaged or broken, resulting in breakage of the suture in short time after implantation or a high fatigue fracture rate after long-term use. When a locking force applied by an operator is insufficient, the suture is not locked by the metal piece, and thus slips off.

In the related art, US7235086B2 discloses a suture locking device, in which a suture that penetrates through an inner cavity of a lock pin is fixed through the lock pin with the hollow inner cavity and a knot locker matched with the lock pin. However, when the locking device of such structure is used to lock the suture, whether the suture is locked or excessively locked cannot be judged effectively.

### SUMMARY

In view of this, for the foregoing shortcoming of the related art, a locking device with a locking feedback function is provided. When the locking device is used to lock a suture in minimally invasive surgery or interventional operation, whether the suture is locked is judged according to a locking feedback to ensure a locking force on the suture, so that the conditions that the suture is not locked and that the suture is excessively locked to be damaged and broken and fail may be prevented, a knot locking effect is ensured, and the success rate of the operation is increased. The specific technical solutions are described below.

A locking device with a locking feedback function is provided. The locking device includes a locking assembly, a handle, and a locking feedback element. The locking assembly is configured to lock a target object. The handle is coupled with the locking assembly and includes a movable portion and a fixed portion. The movable portion is configured to move relative to the fixed portion to drive the locking assembly to lock the target object. The locking feedback element is movably disposed in the fixed portion and configured to cooperate with the movable portion of the handle to feed back whether the target object is locked.

A heart valve repair system is further provided. The heart valve repair system includes the locking device provided in any of above-mentioned implementations and at least one suture. The suture is configured to be implanted to a valve leaflet of a heart valve.

Compared with the related art, the disclosure at least has the following beneficial effects. When the locking device with the locking feedback function of the disclosure is used to lock the target object, whether the target object is locked by the locking assembly may be judged through the locking feedback element to ensure a locking degree of the target object, so that a locking effect is ensured, and the success rate of the operation is increased. According to the heart valve repair system provided in the disclosure, the suture is implanted to one or more valve leaflets of the heart valve at first, and then the suture is fixed to the apex of the heart, the papillary muscle, or the ventricular wall through the locking device, or multiple sutures are fixed together through the locking device. In a locking process, whether the suture is locked may be judged through the locking feedback element to ensure the locking degree of the suture, so that the locking effect is ensured, and the success rate of the operation is increased. Moreover, the suture may be prevented from being damaged or broken by excessive locking, and the suture may be avoided from being broken.

### BRIEF DESCRIPTION OF THE DRAWINGS

In order to describe the technical solutions in the implementations of the disclosure more clearly, the drawings required to be used in the implementations will be simply introduced below. It is apparent that the drawings described below are merely some implementations of the disclosure. Other drawings may further be obtained by those of ordinary skill in the art according to these drawings without creative work.
FIG. 1 is a structure diagram of a locking device with a locking feedback function according to a first implementation of the disclosure.
FIG. 2 is an internal sectional view of the locking device with the locking feedback function according to the first implementation.
FIG. 3 is a three-dimensional structural exploded view of a locking feedback element and a fixed portion according to the first implementation.
FIG. 4 is a back view of the locking feedback element in FIG. 3.
FIG. 5a is a left view of the locking feedback element in FIG. 3.
FIG. 5b is a sectional view along line A-A in FIG. 5a.
FIG. 6 is a partial enlarged view of part M in FIG. 3.
FIG. 7 is a partial enlarged view of part N in FIG. 2.
FIG. 8 is a structure diagram of a locking feedback element in a locking device with a locking feedback function according to a second implementation of the disclosure.
FIG. 9 is a back view of FIG. 8.
FIG. 10a is a left view of FIG. 8.
FIG. 10b is a sectional view along line A-A in FIG. 10a.
FIG. 11 is a schematic diagram of a state of the locking feedback element after locking of the locking device according to the second implementation.
FIG. 12a is a schematic diagram of an initial state of the locking feedback element in a fixed portion according to the second implementation.
FIG. 12b is a partial enlarged view of part S in FIG. 12a.
FIG. 12c is a schematic diagram of a state of the locking feedback element in the fixed portion after locking feedback according to the second implementation.
FIG. 12d is a partial enlarged view of part T in FIG. 12c.
FIG. 13a and FIG. 13b are structure diagrams of a movable portion in a locking device with a locking feedback function according to a third implementation of the disclosure.
FIG. 14 is a structure diagram of a locking device with a locking feedback function according to a fourth implementation of the disclosure.
FIG. 15 is a partial enlarged view of part R in FIG. 14.
FIG. 16a is a schematic diagram of the locking device in FIG. 1 before locking feedback.
FIG. 16b is an internal structure diagram of the locking device in FIG. 16a.
FIG. 17a is a schematic diagram of the locking device in FIG. 1 after locking feedback.
FIG. 17b is an internal structure diagram of FIG. 17a.
FIG. 18 is a partial enlarged view of part W in FIG. 17b.
FIG. 19 is an exploded view of a locking assembly of the locking device according to the disclosure.
FIG. 20a is a schematic view illustrating that a suture is not fixed by a lock pin according to the disclosure.
FIG. 20b is a schematic view illustrating that the suture is fixed by the lock pin according to the disclosure.
FIGS. 21a -21c are schematic structural views illustrating a handle illustrated in FIG. 19.
FIGS. 22a -22b are schematic structural views illustrating a bunching device of the locking assembly according to the disclosure.
FIG. 23 is a schematic structural view illustrating a suture fixation illustrated in FIG. 22.
FIG. 24 is an enlarged view of portion A of FIG. 21c.
FIGS. 25a -25b are schematic structural views illustrating a handle of a locking device according to a fifth implementation of the disclosure, where FIG. 25b is a cross-sectional view of the handle illustrated in FIG. 25a, taken along line B-B.
FIG. 26 is an enlarged view of portion B of FIG. 25b.
FIG. 27 is an exploded view of a locking device according to a sixth implementation of the disclosure.
FIG. 28 is a schematic structural view illustrating an adjusting device illustrated in FIG. 27.
FIG. 29 is a perspective view illustrating the lock pin according to the disclosure.
FIG. 30 is a perspective view illustrating a lock pin according to another implementation of the disclosure.
FIG. 31 is a schematic structural view illustrating a squeezing assembly according to the disclosure.
FIGS. 32a -32b are schematic views illustrating holding and pressing the lock pin by the locking device according to the disclosure.
FIGS. 33a -33b are enlarged views of a portion C illustrated in FIG. 32b.
FIGS. 34a -34b are schematic structural views illustrating a holding portion of the locking device according to the disclosure.

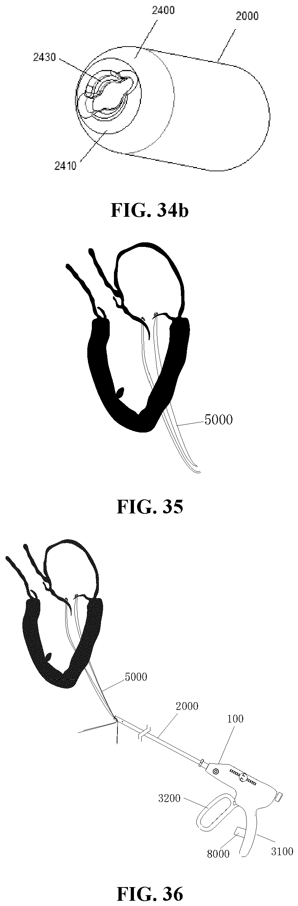

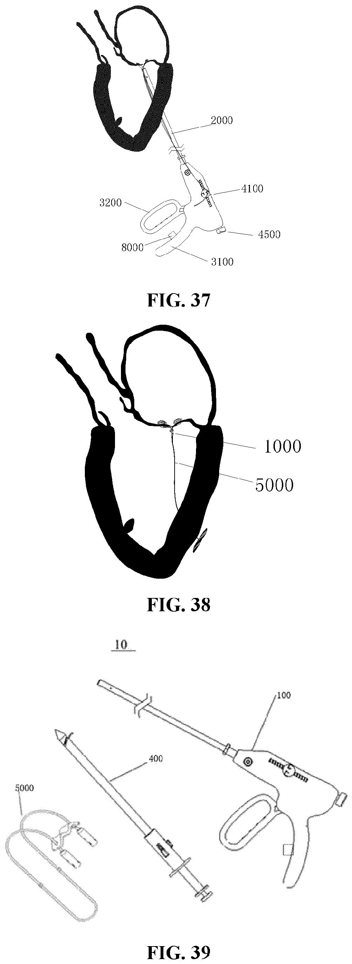

FIGS. 35-38 are schematic views illustrating a process of repairing leaflets of a mitral valve by the locking device provided in the sixth implementation, where FIG. 35 is a schematic view illustrating implanting sutures into an anterior leaflet and a posterior leaflet, FIG. 36 is a schematic view illustrating that the sutures pass through the locking device according to the disclosure, FIG. 37 is a schematic view illustrating that the locking device is operated to adjust lengths of the sutures and hold and press the lock pin according to the disclosure, FIG. 38 is a schematic view illustrating that proximal ends of the sutures are fixed to a ventricular wall through the lock pin according to the disclosure.
FIG. 39 is a schematic structural view illustrating a heart valve repair system according to the disclosure.

### DETAILED DESCRIPTION

The technical solutions in the implementations of the disclosure will be clearly and completely described below in combination with the drawings in the implementations of the disclosure. It is apparent that the described implementations are not all but merely part of implementations of the disclosure. All other implementations obtained by those of ordinary skill in the art based on the implementations in the disclosure without creative work shall fall within the scope of protection of the disclosure.

Orientation definition: the following defines an orientation close to an operator as a proximal end and an orientation away from the operator as a distal end. Unless otherwise defined, all technological and scientific terms used in the disclosure have meanings the same as those usually understood by those skilled in the art of the disclosure. Related terms used in the specification of the disclosure are only for a purpose of describing specific implementations, and cannot be understood as limits to the disclosure.

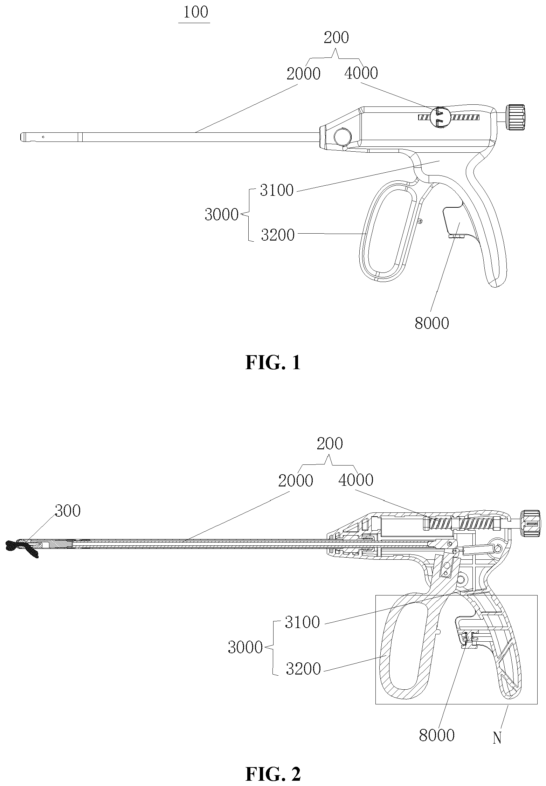

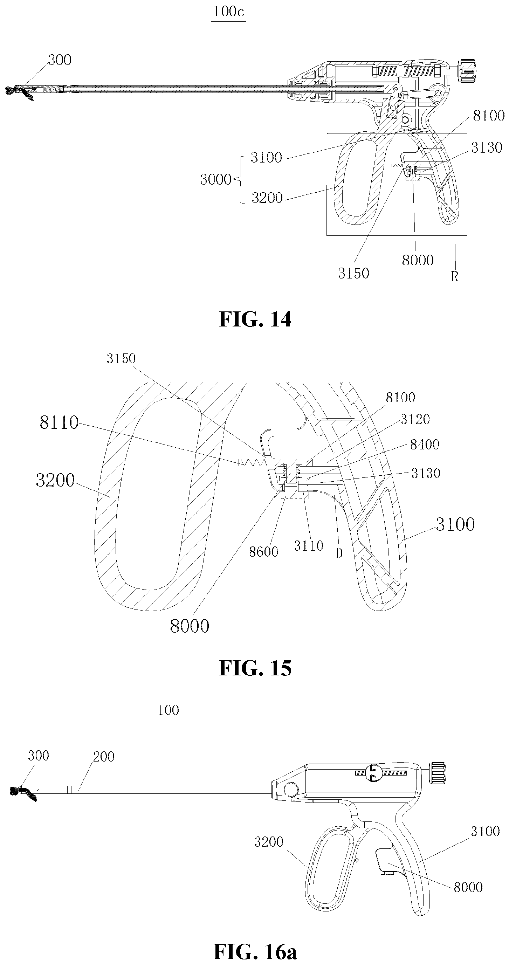

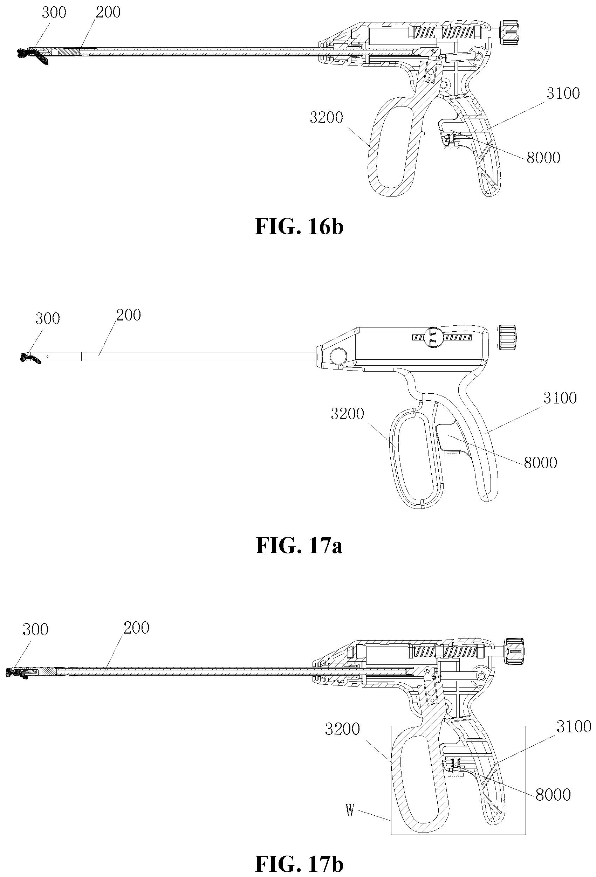

Referring to FIG. 1 and FIG. 2, a locking device 100 with a locking feedback function is provided. The locking device 100 includes a locking assembly 200, a handle 3000, and a locking feedback element 8000. The locking assembly 200 is configured to lock a target object 300 (referring to FIG. 2). The locking assembly 200 is coupled with the handle 3000. The handle 3000 includes a fixed portion 3100 and a movable portion 3200. The movable portion 3200 is configured to move relative to the fixed portion 3100 to drive the locking assembly 200 to lock the target object 300 (referring to FIG. 16a to FIG. 17b). The locking feedback element 8000 is movably disposed in the fixed portion 3100, and is configured to cooperate with the movable portion 3200 of the handle 3000 to feed back whether the target object 300 is locked. The movable portion 3200 of the handle 3000 moves relative to the fixed portion 3100 to further drive the locking assembly 200 to lock the target object 300. As such, an operator applies an acting force to the locking feedback element 8000 at the same time of operating the movable portion 3200 and the fixed portion 3100, such that a feedback effect of the locking feedback element 8000 is synchronized with an operation of the handle 3000, a locking degree of the target object 300 by the locking assembly 200 can be fed back accurately.

When the locking device 100 with the locking feedback function in the disclosure is used to lock the target object 300, whether the target object 300 is locked by the locking assembly 200 may be judged through the locking feedback element 8000 to ensure the locking degree of the target object 300, so that a locking effect is ensured, and the success rate of the operation is increased.

In a further implementation, the locking feedback element 8000 is operable to be exposed outside the fixed portion 3100 or impact with the fixed portion 3100 to make a sound, to feed back whether the target object 300 is locked. When the locking assembly 200 locks the target object 300, the locking feedback element 8000 impacts with the fixed portion 3100 to make a sound to prompt the operator that the target object 300 has reached a target locking degree, and in such case, a locking operation is completed. Feeding back through a sound made by impacting between the locking feedback element 8000 and the fixed portion 3100 ensures timelier judgment, enables the operator to learn about a locking state without lowering the head to view the locking device 100, and is more favorable for the operator to observe a medical imaging device such as ultrasonic equipment during operation. In case of feeding back through a sound, a portion, impacting with the fixed portion 3100, of the locking feedback element 8000 is made of a relatively hard material, so that a clear impact sound may be made by impacting. In other implementations, whether the target object 300 is locked may also be fed back by protrusion of a partial unit in the locking feedback element 8000 from an initial position or using a bouncing element.

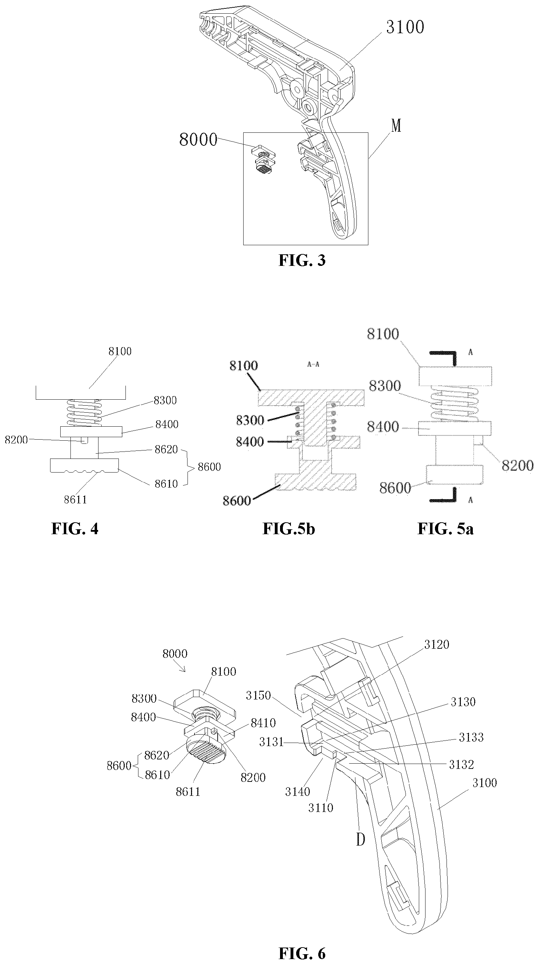

Referring to FIG. 3 to FIG. 7, in a further implementation, the locking feedback element 8000 includes a feedback sliding block 8100, a supporting element 8200, and an elastic element 8300 coupled between the feedback sliding block 8100 and the supporting element 8200 (referring to FIG. 6). The feedback sliding block 8100 is operable to move toward a proximal end of the fixed portion 3100 under an action of the movable portion 3200. The supporting element 8200 is capable of moving with the feedback sliding block 8100. The fixed portion 3100 defines a first notch 3110 thereon. When the supporting element 8200 moves to and falls in the first notch 3110, the locking feedback element 8000 is exposed outside the fixed portion 3100 or impacts with the fixed portion 3100 to make a sound, to feed back that the target object 300 is locked. The elastic element 8300 is used to, when the supporting element 8200 moves to the first notch 3110, return to a natural state to make the supporting element 8200 fall in the first notch (referring to FIG. 6). It is to be understood that, when the supporting element 8200 does not reach the first notch 3110, the elastic element 8300 is in a compressed state. It is to be understood that the first notch 3110 may be formed in a housing surface of the fixed portion 3100.

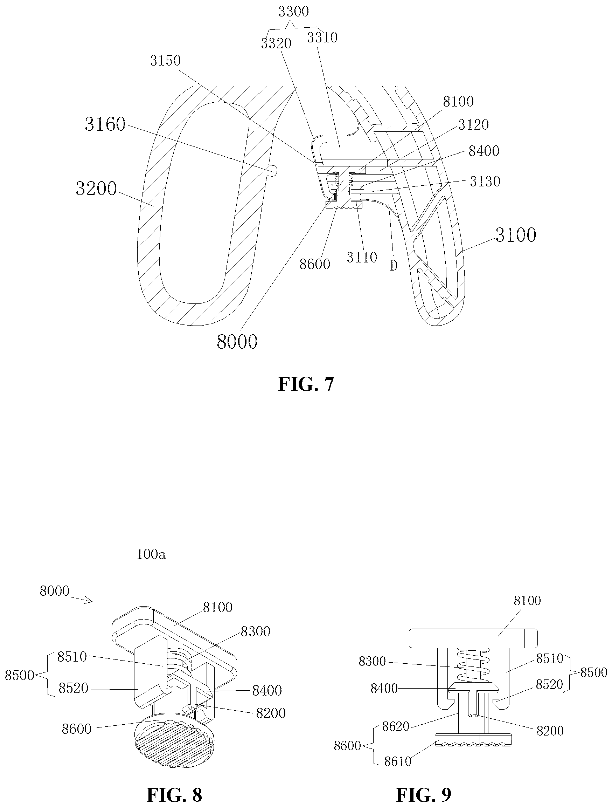

Referring to FIG. 6 and FIG. 7, in a further implementation, the fixed portion 3100 defines a first limiting groove 3120 and a second limiting groove 3130 therein. In an implementation, the fixed portion 3100 includes a mounting frame 3300 protruding to the movable portion 3200 (referring to FIG. 7). The mounting frame 3300 includes an extending plate 3310 and a flange 3320 arranged at an edge of the extending plate 3310. The first limiting groove 3120 and the second limiting groove 3230 are formed in parallel at an interval in the extending plate 3310. Each of the first limiting groove 3120 and the second limiting groove 3130 extends along a direction from a distal end to the proximal end. The feedback sliding block 8100 can move toward the proximal end of the fixed portion 3100 in the first limiting groove 3120 under the action of the movable portion 3200. The supporting element 8200 can move in the second limiting groove 3130 toward the same direction as the feedback sliding block 8100. The first notch 3110 is formed in an edge adjacent to the second limiting groove 3130. The first notch 3110 is in communication with the second limiting groove 3130. The first notch 3110 is formed adjacent to the proximal end of the fixed portion 3110, and extends through an outer surface of a bottom end D of the fixed portion 3100. When being moved to face the first notch 3110, the supporting element 8200 will fall in the first notch 3110 under the action of the elastic element 8300.

The bottom end D of the fixed portion 3100 refers to an end portion in a direction perpendicular to a direction from the proximal end to the distal end of the locking device 100. That is, in the implementation, the locking feedback element 8000 moves along a direction from the distal end to the proximal end of the fixed portion 3100 in the fixed portion 3100 under the action of the movable portion 3200. When the supporting element 8200 moves to the first notch 3110, the supporting element 8200 will fall in the first notch 3110 towards the bottom end D of the fixed portion 3100. It is to be understood that a length of the supporting element 8200 may be set to ensure that the supporting element 8200 will exposed outside the outer surface of the bottom end D of the fixed portion 3100 through the first notch 3110 when falling in the first notch 3110. In such case, when the operator sees the part of the supporting element 8220 that exposes from the first notch 3110, the operator is prompted that the locking device 100 has locked the target object 300.

In a further implementation, the locking feedback element 8000 further includes an impact element 8400. The elastic element 8300 is elastically abutted against the impact element 8400. The supporting element 8200 is disposed on a side of the impact element 8400 away from the elastic element 8300. When the supporting element 8200 is moved to face the first notch 3110, the elastic element 8300 drives the impact element 8400 to impact with the fixed portion 3100 to make a sound to feed back that the target object 300 has been locked, and the supporting element 8200 falls in the first notch 3110. It is to be understood that the supporting element 8200 and the impact element 8400 may be connected through a buckle, a screw, or an adhesive. Alternatively, the supporting element 8200 and the impact element 8400 may be integrally formed. When the supporting element 8200 falls in the first notch 3110, the impact element 8400 may be pressed by the elastic element 8300 to be abutted against the bottom end D of the fixed portion 3100. Since the impact element 8400 is arranged above the supporting element 8200, when the supporting element 8200 falls in the first notch 3110 under the action of the elastic element 8300, the elastic element 8300 also applies an acting force to the impact element 8400 to cause the impact element 8400 impacts an upper surface of the bottom end D of the fixed portion 3100 and be positioned in the fixed portion 3100. When a portion, impacting with the fixed portion 3100, of the impact element 8400 is supported by a hard material, a sound may be made, and the operator is prompted, through the sound made by impacting, that the target object 300 has been locked. It is to be understood that the impact element 8400 may be clamped to a corresponding position according to the structure of the fixed portion 3100, namely the impact element 8400 may be clamped to various positions.

In a further implementation, a cross sectional area of the impact element 8400 is larger than a cross sectional area of the first notch 3110, such that the impact element 8400 may be abutted against a part above the first notch 3110 when the supporting element 8200 falls in the first notch 3110.

Referring back to FIG. 6, in a further implementation, the second limiting groove 3130 includes a first supporting surface 3131. The supporting element 8200 is operable to slide on the first supporting surface 3131. The first notch 3110 extends through the first supporting surface 3131. The first notch 3110 is formed adjacent to the proximal end of the fixed portion 3110.

When the supporting element 8200 falls in the first notch 3110, the impact element 8400 is positioned on the first supporting surface 3131 around the first notch 3110. It is to be understood that the first supporting surface 3113 is a supporting surface in a distal direction of the first notch 3110. If both the first supporting surface 3131 and the impact element 8400 are made of a hard material, a sound may be made when the impact element 8400 is driven by the elastic element 8300 to impact on the first supporting surface 3131. The impact element 8400 includes an impact surface 8410 (referring to FIG. 6). The impact surface 8410 is a surface, away from the elastic element 8300, of the impact element 8400. The impact surface 8410 is configured to impact with the fixed portion 3100. In the implementation, the impact surface 8410 is configured to impact with the first supporting surface 3131.

In a further implementation, the second limiting groove 3130 further includes a second supporting surface 3132. The second supporting surface 3132 and the first supporting surface 3131 are arranged on two sides of the first notch 3110. The second supporting surface 3132 and the first supporting surface 3131 are on the same horizontal plane. When the supporting element 8200 falls in the first notch 3110, the impact element 8400 is clamped to part of the first supporting surface 3131 and part of the second supporting surface 3132 on the two sides of the first notch 3110. In the implementation, the impact element 8400 simultaneously impacts the first supporting surface 3131 and the second supporting surface 3132, and the impact element 8400 is supported by the two supporting surfaces, so that a volume of the sound made by impacting is increased, and meanwhile, the impact element 8400 may further be prevented from being separated from the first notch 3110.

It is to be understood that the second limiting groove 3130 may further include a third supporting surface 3133 (referring to FIG. 6), the third supporting surface 3133 is arranged between the first supporting surface 3131 and the second supporting surface 3232. The three supporting surfaces, i.e., the third supporting surface 3133, the first supporting surface 3131, and the second supporting surface 3132, are all configured to impact with the impact surface 8410 in the impact element 8400 to make a louder impact sound.

In a further implementation, the locking feedback element 8000 further includes a feedback button 8600 (referring to FIG. 4 or FIG. 9). The feedback button 8600 includes a button portion 8610 and a second connecting portion 8620. The button portion 8610 is coupled to the impact element 8400 via the second connecting portion 8620, and the button portion 8610 is exposed outside the outer surface of the bottom end D of the fixed portion 3100 (referring to FIG. 6 and FIG. 7). When the supporting element 8200 falls in the first notch 3110, the impact element 8400 moves to a bottom of the fixed portion 3100 under the action of the elastic element 8300, and simultaneously drives the button portion 8610 on the feedback button 8600 to move. Since the button portion 8610 is exposed outside the outer surface of the bottom end D of the fixed portion 3100, the operator may observe a relative position distance of the button portion 8610 and the bottom end D of the fixed portion 3100 to judge whether the target object 300 is locked to obtain a feedback about the locking state. It is to be understood that the button portion 8610 may be arranged in a relatively bright color or a shape easy to observe. In the implementation, the locking state may also be fed back through a sound.

Referring back to FIG. 6, in a further implementation, the fixed portion 3100 further includes a second notch 3140. The second notch 3140 extends along the second limiting groove 3130. The second notch 3140 extends through the second limiting groove 3130 and the outer surface of the bottom end D of the fixed portion 3100. The second connecting portion 8620 extends from the second notch 3140. When the feedback sliding block 8100 slides in the first limiting groove 3110, the second connecting portion 8620 moves in the second notch 3140 to drive the button portion 8610 exposed outside the bottom end D of the fixed portion 3100 to move. A size of the second notch 3140 is adapted to the second connecting portion 8620. In other implementations, the second notch 3140 may also be formed in a lateral surface of the fixed portion 3100.

In a further implementation, when the supporting element 8200 is abutted against the first supporting surface 3131 in the second limiting groove 3130, a distance between the button portion 8610 and the outer surface, away from the second limiting groove 3130, of the fixed portion 3100 is less than a preset distance. That is, a distance between the button portion 8610 and the surface of the bottom end D of the fixed portion 3100 is less than the preset distance. Preferably, the preset distance is zero, namely the button portion 8610 is attached to the surface of the bottom end D of the fixed portion 3100.

In a further implementation, after the supporting element 8200 falls in the first notch 3110, it is also possible to apply a pressing force on the feedback button 8600 to press the feedback sliding block 8100 until the supporting element 8200 is located in the second limiting groove 3130, and then apply a pushing force on the feedback button 8600 to enable the feedback button 8600 to move toward the distal end of the fixed portion 3100, such that the feedback button 8600, the supporting element 8200, and the feedback sliding block 8100 return. After returning, the locking feedback element 8000 can be operable to feed back whether the target object 300 is locked again. Therefore, the locking device 100 may be repeatedly used in an operating process.

In a further implementation, an indented portion 8611 (referring to FIG. 4) or a protruding portion is formed on the surface, away from the second connecting portion 8620, of the button portion 8610. The intended portion 8611 or protruding portion formed on the button portion 8610 may increase a frictional force between the operator and the button portion 8610, so that the feedback button 8600 may be operated faster and more conveniently to make the supporting element 8200 and the feedback sliding block 8100 return.

Referring back to FIG. 6 and FIG. 7, in a further implementation, the fixed portion 3100 further defines a third notch 3150 therein. The third notch 3150 extends in a direction which is consistent with a moving direction of the feedback sliding block 8100. The movable portion 3200 is configured to drive the feedback sliding block 8100 to move via the third notch 3150. An opening size of the third notch 3150 is set according to practical requirements.

In a further implementation, a lug boss 3160 (referring to FIG. 7) is arranged at a position, corresponding to the third notch 3150, of the movable portion 3200. When the movable portion 3200 moves to the fixed portion 3100, the lug boss 3160 is abutted against the feedback sliding block 8100, and drives the feedback sliding block 8100 to move in the first limiting groove 3120. The lug boss 3160 is configured to apply an acting force to the feedback sliding block 8100, so that the feedback sliding block 8100 can be driven more effectively.

In a further implementation, the elastic element 8300 includes one of a spring, an elastic piece, or a bouncy ball. In FIG. 4 and FIG. 9, schematic diagrams of the elastic element 8300 that is a spring are shown.

For better describing the technical solution of the disclosure, a working process of the disclosure will be described below taking a first implementation as an example.

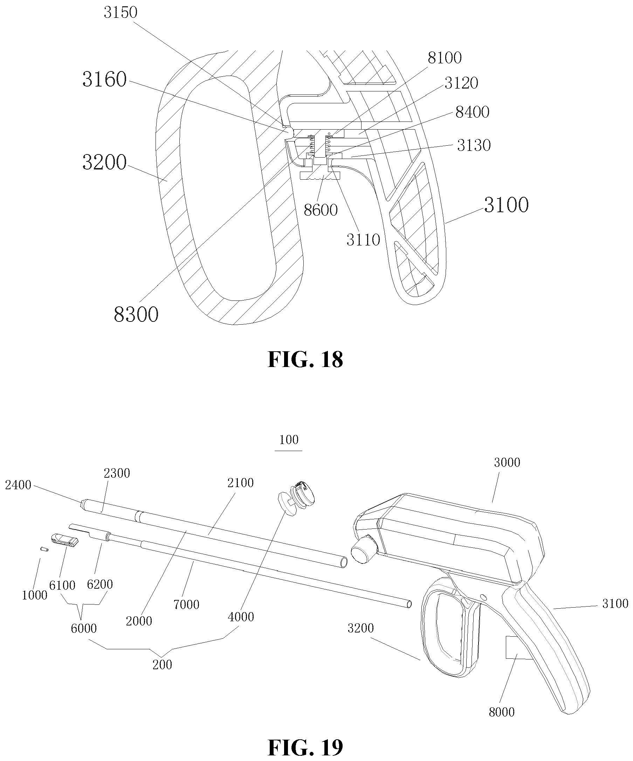

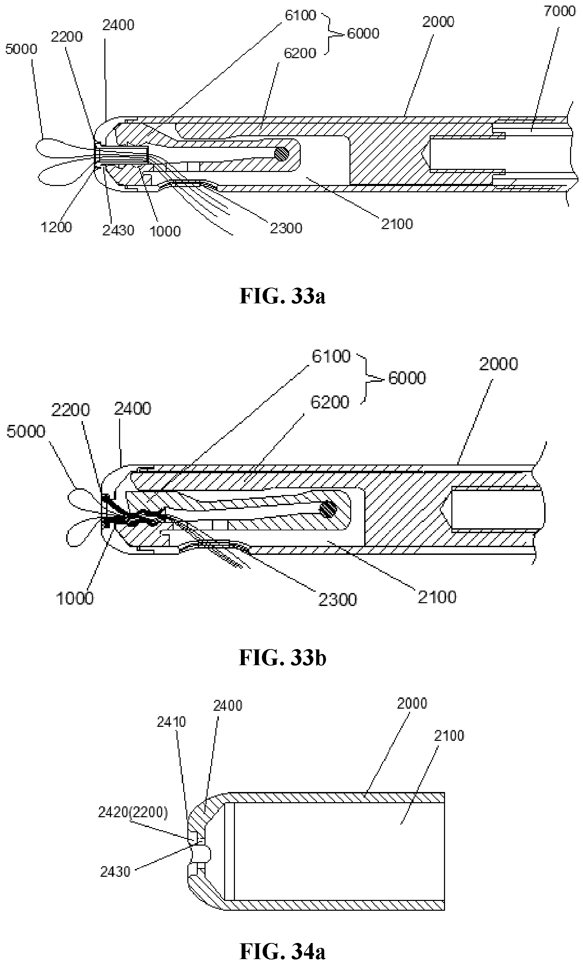

Referring to FIG. 16a to FIG. 18, in the implementation, the locking assembly 200 clamps the target object 300 using a chuck 6100 (referring to FIG. 33a and FIG. 33b). The target object 300 is a lock pin 1000 and multiple sutures 5000 that penetrates through the lock pin 1000. Referring to FIG. 16a, FIG. 16b, FIG. 6, and FIG. 7, when the locking assembly 200 does not lock the target object 300, the movable portion 3200 and the fixed portion 3100 of the handle 3000 are in a separated state. In such case, the supporting element 8200 in the locking feedback element 8000 is located in the second limiting groove 3130, and the elastic element 8300 is in the compressed state (referring to FIG. 6 and FIG. 7). Referring to FIG. 17a, FIG. 17b, and FIG. 18, when the locking assembly 200 locks the target object 300, the movable portion 3200 moves to the fixed portion 3100, the lug boss 3160 on the movable portion 3200 abuts against the feedback sliding block 8100 to move in the first limiting groove 3120 to drive the whole locking feedback element 8000 to move toward the proximal end of the fixed portion 3100. The supporting element 8200 moves in the second limiting groove 3130. When the supporting element 8200 is moved to face the first notch 3110, the elastic element 8300 returns to an initial state and applies a force to the supporting element 8200, so as to enable the supporting element 8200 to fall in the first notch 3110, and at this time, the impact element 8400 will impact with the second limiting groove 3130 to make a sound to prompt the operator that the target object 300 has been locked, namely the multiple sutures 5000 have been fixed in the lock pin 1000.

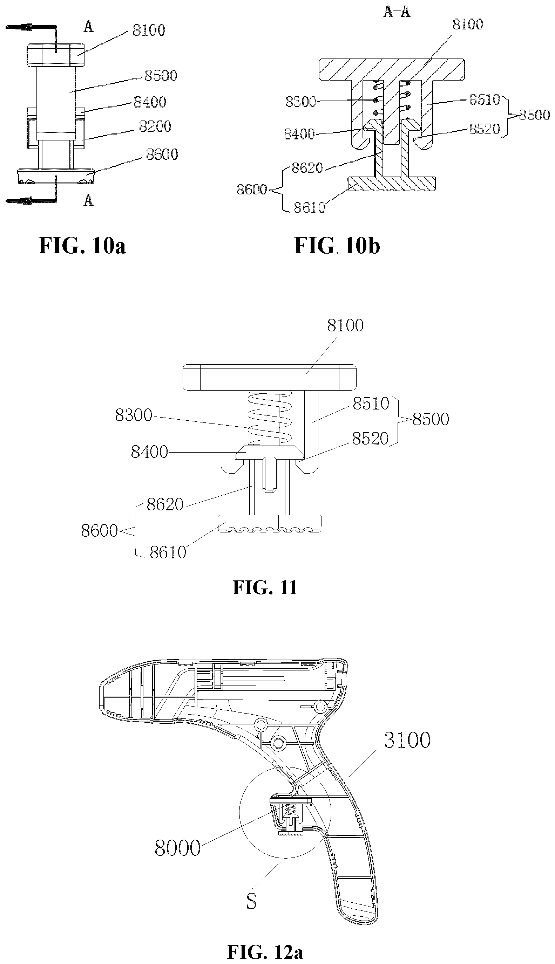

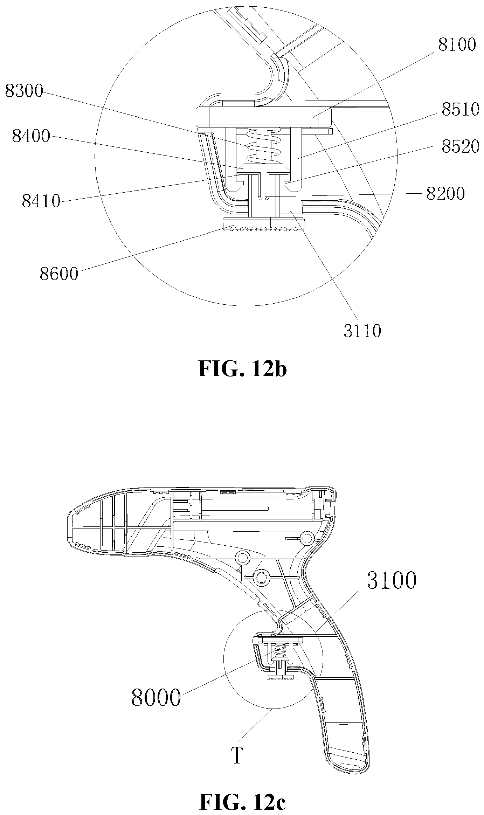

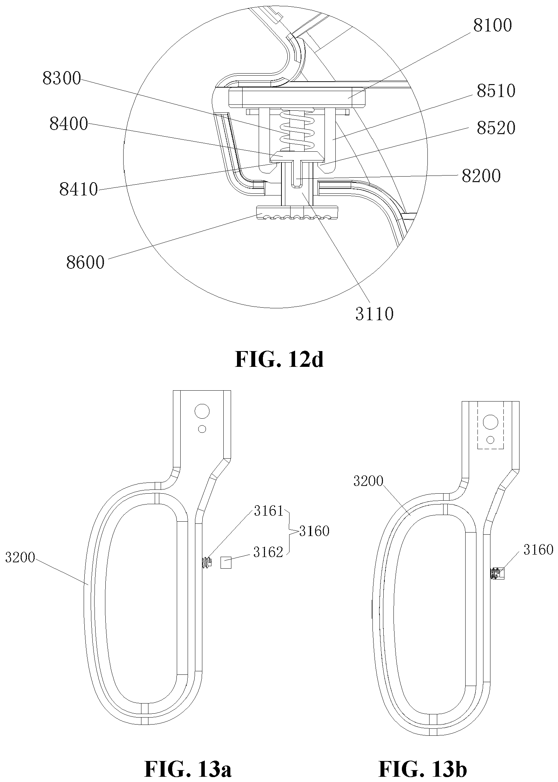

Referring to FIG. 8 to FIG. 12d, a locking device 100a with a locking feedback function is provided according to a second implementation of the disclosure. In the locking device 100a, the locking feedback element 8000 further includes at least one inverted buckle 8500. The inverted buckle 8500 includes a first connecting portion 8510 and a clamping portion 8520. The clamping portion 8520 is coupled to the feedback sliding block 8100 via the first connecting portion 8510. The clamping portion 8520 is disposed below the feedback sliding block. An orthographic projection of the impact element 8400 on the feedback sliding block 8100 is at least partially overlapped with an orthographic projection of the clamping portion 8520 on the feedback sliding block 8100. When the supporting element 8200 falls in the first notch 3110, the impact element 8400 is abutted against the clamping portion 8520 (referring to FIG. 11). In the implementation, when the supporting element 8200 falls in the first notch 3110, the elastic element 8300 simultaneously acts on the impact element 8400 to impact on the clamping portion 8520 to make a sound, namely the impact surface 8410 of the impact element 8400 impacts on the clamping portion 8520 (referring to FIG. 12d). The orthographic projections of the impact element 8400 and the clamping portion 8520 on the feedback sliding block 8100 are arranged to be at least partially overlapping to ensure that the impact element 8400 is clamped to the clamping portion 8520.

In a further implementation, there are two inverted buckles 8500 arranged on the two opposite sides of the impact element 8400. When the supporting element falls in the first notch 3110, the impact element 8400 is abutted against the clamping portions 8520 of the two inverted buckles 8500. In some other implementations, there may be multiple inverted buckles 8500.

Referring to FIG. 13a and FIG. 13b, a locking device with a locking feedback function is provided according to a third implementation of the disclosure. FIG. 13a and FIG. 13b only show the movable portion 3200, and the other parts are the same as those in the first implementation. Another lug boss 3160 is arranged on the movable portion 3200 of the handle 3000. In the implementation, the lug boss 3160 includes a first junction portion 3161 and a second junction portion 3162, and the first junction port 3161 and the second junction portion 3162 are operable to extend and retract relative to each other to adjust an axial length of the lug boss 3160. The axial length of the lug boss 3160 may be adjusted through the first junction portion 3161 and the second junction portion 3162 to feed back different locking effects. When the target object 300 is the lock pin 1000 and the sutures 5000, a magnitude of a locking force may be adjusted more favorably for different specifications of sutures 5000 or different numbers of sutures 5000 with the same specification, and for different specifications of lock pins 1000 (i.e., lock pins 1000 with the same outer diameter. Since different inner diameters cause different wall thicknesses of the lock pins 1000, different pressure gripping forces for locking are different). For example, when the number of the sutures 5000 in the lock pin 1000 increases or the wall thickness of the lock pin 1000 increases, the chuck 6100 (referring to FIG. 33a and FIG. 33b) may press and grip the lock pin 1000 with a relatively a weak force, and when the lock pin 1000 is deformed, the second junction portion 3162 may be adjusted to increase the total axial length of the lug boss 3160 to shorten a stroke when a locking feedback state is reached.

In a further implementation, the first junction portion 3161 and the second junction portion 3162 are operable to extend and retract relative to each other in a threaded extension and retraction manner or a buckle extension and retraction manner.

In a further implementation, the first junction portion 3161 and the second junction portion 3162 are operable to extend and retract relative to each other in the threaded extension and retraction manner. The first junction portion 3161 is one of a screw element and a screw cap adapted to the screw element, and the second junction portion 3162 is the other of the screw element and the screw cap. In the implementation, the first junction portion 3161 uses the screw element, and the second junction portion 3162 uses the screw cap. The screw element is arranged on the movable portion 3200, and the screw cap rotates relative to the screw element to adjust the axial length of the lug boss 3160. In other implementations, the screw cap may be arranged on the movable portion 3200, and the screw element rotates relatively to adjust the axial length of the lug boss 3160.

Referring to FIG. 14 and FIG. 15, a locking device 100c with a locking feedback function is provided according to a fourth implementation of the disclosure. In the locking device 100c, when the supporting element 8200 is located in the second limiting groove 3130, the feedback sliding block 8100 extends outside the outer surface of the fixed portion 3100 through the third notch 3150. That is, in the implementation, the feedback sliding block 8100 is relatively long. When moving toward the fixed portion 3100, the movable portion 3200 may be directly abutted against the part, extending outside the outer surface of the fixed portion 3100, of the feedback sliding block 8100 to further drive the whole feedback sliding block 8100 to move toward the proximal end of the fixed portion 3100.

In a further implementation, a length-adjustable telescopic portion 8110 is further disposed at an end, extending outside the outer surface of the fixed portion 3100 from the third notch 3150, of the feedback sliding block 8100. As illustrated in FIG. 13a, the axial length of the lug boss 3160 is adjusted through the first junction portion 3161 and second junction portion 3162 in the lug boss 3160 to further adjust the magnitude of the locking force. In the implementation, the telescopic portion 8110 is used to adjust the magnitude of the locking force. It is to be understood that the telescopic portion 8110 may extend and retract in the threaded extension and retraction or buckle extension and retraction manner.

It is to be understood that a working principle of other implementations of the disclosure is similar to that of the first implementation, and will not be repeated herein.

Structural features of the target object 300 and the locking assembly 200 of the disclosure, as well as specific implementations of locking the target object 300 by the locking assembly 200, are described below based on the above-mentioned implementations.

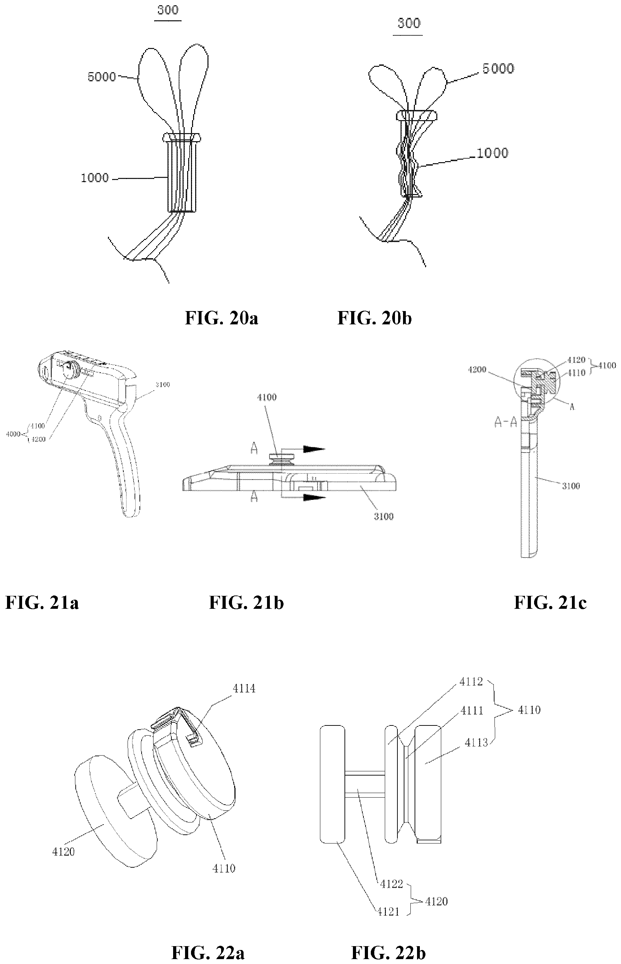

Referring to FIGS. 19, 20a, and 20b, based on the first implementation, the target object 300 in this implementation includes a lock pin 1000 and a suture 5000. The lock pin 1000 is used to accommodate or fix the suture 5000 (referring to FIG. 20a or FIG. 20b, where FIG. 20a illustrates that the suture 5000 is not fixed by the lock pin 1000, and FIG. 20b illustrates that the suture 5000 is fixed by the lock pin 1000). The locking assembly 200 is used to lock the suture 5000 in the lock pin 100. The locking assembly 200 includes an outer tube 2000 and an adjusting device 4000. The outer tube 2000 defines a receiving cavity 2100 therein, and the lock pin 1000 is disposed at a distal end of the receiving cavity 2100 (as illustrated in FIG. 33a and FIG. 33b). A fixed portion 3100 is coupled to a proximal end of the outer tube 2000. The adjusting device 4000 is disposed on the fixed portion 3100. The adjusting device 4000 is coupled to the proximal end of the suture 5000 and used for adjusting tightening or loosening of the suture 5000. It is to be understood that the adjusting device 4000 may be any device capable of adjusting the tightening or loosening of the suture 5000, such as a bunching device or a buckle. It is to be understood that the number of the adjusting device 4000 can be set according to need. Preferably, two adjusting devices 4000 are respectively disposed on two opposite sides of the fixed portion 3100 for adjusting two sets of sutures 5000 respectively.

As illustrated in FIGS. 21a -21c, in a further implementation, the adjusting device 4000 includes a bunching device 4100 movably coupled to the fixed portion 3100. The bunching device 4100 is coupled to the proximal end of the suture 5000 to adjust the tightening or loosening of the suture 5000. By adjusting the tightening or loosening of the suture 5000 through the bunching device 4100, the length of the suture 5000 can be effectively controlled, and a slight adjustment of the length of the suture 5000 can be achieved. For example, the suture 5000 is wound on the bunching device 4100, and the suture 5000 is tightened or loosened by changing a winding direction or a winding manner.

The adjusting device 4000 further includes an adjusting rail 4200 disposed on the fixed portion 3100. The bunching device 4100 is coupled to the fixed portion 3100 via the adjusting rail 4200. The bunching device 4100 can slide back and forth on the adjusting rail 4200 along an axial direction of the adjusting rail 4200, thereby adjusting the tightening or loosening of the suture 5000. It is to be understood that the bunching device 4100 adjusts the tightening or loosening of the suture 5000 when rolling on the adjusting rail 4200. It is to be understood that the axial direction of the adjusting rail 4200 coincides with the axial direction of the whole locking device 100, or the axial direction of the adjusting rail 4200 and the axial direction of the whole locking device 100 define an angle of preset degrees, so as to allow the bunching device 4100 to roll on the adjusting rail 4200, thereby adjusting the tightening or loosening of the suture 5000. The axial direction of the whole locking device 100 refers to a direction from the proximal end to the distal end.

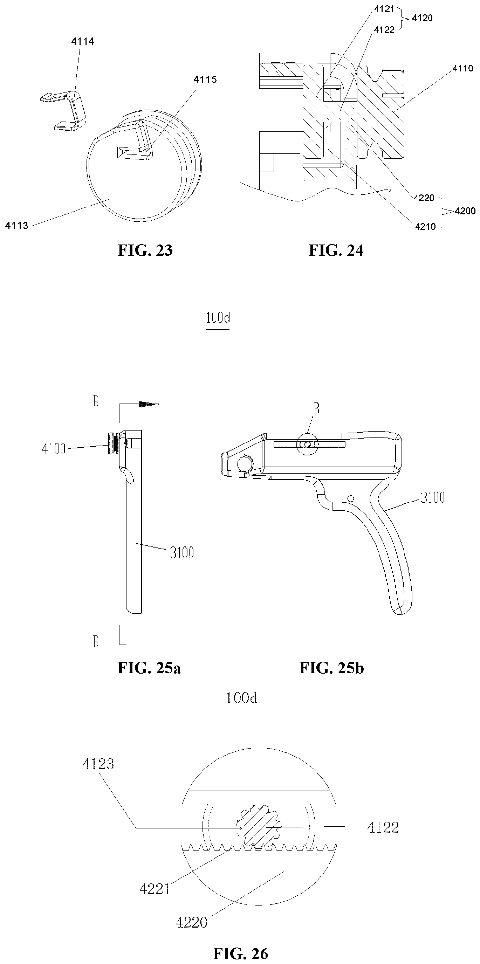

As illustrated in FIG. 22a and FIG. 22b, in a further implementation, the bunching device 4100 includes a suture fixation 4110 and a handle connecting portion 4120 coupled with the suture fixation 4110. The suture fixation 4110 is detachably coupled to the suture 5000. The suture 5000 can be fixed to the suture fixation 4110 by winding, pressing, fastening, or the like. The handle connecting portion 4120 is disposed in the adjusting rail 4200 (as illustrated in FIG. 24). The bunching device 4100 can move back and forth on the adjusting rail 4200 along the axial direction of the adjusting rail 4200 via the handle connecting portion 4120. The handle connecting portion 4120 is disposed in the adjusting rail 4200 by clamping or the like to ensure that the bunching device 4100 cannot slip off the handle 3000.

As illustrated in FIG. 22b, in a further implementation, the suture fixation 4110 includes a spool 4111, a first blocking portion 4112, and a second blocking portion 4113. The first blocking portion 4112 and the second blocking portion 4113 are respectively coupled to two opposite ends of the spool 4111. The second blocking portion 4113 is further away from the handle connecting portion 4120 than the first blocking portion 4112. When the suture 5000 is wound on the spool 4111, the first blocking portion 4112 and the second blocking portion 4113 at two ends of the spool 4111 can effectively prevent the suture 5000 from slipping off the spool 4111. It is to be understood that the first blocking portion 4112 and the second blocking portion 4113 each have a larger diameter than the spool 4111.

Referring to FIG. 22a, in a further implementation, the suture fixation 4110 further includes an elastic sheet 4114. The elastic sheet 4114 is fixed to the second blocking portion 4113. The elastic sheet 4114 is used to fix the suture 5000. Referring to FIG. 23, it is to be understood that the second blocking portion 4113 defines an elastic sheet receiving groove 4115. The elastic sheet 4114 is received in the elastic sheet receiving groove 4115. After the suture 5000 is wound on the spool 4111 several times, the proximal end of the suture 5000 is fixed to the elastic sheet 4114 and received in the elastic sheet receiving groove 4115 to fix the proximal end of the suture 5000 to the bunching device 4100. It is to be understood that the elastic sheet receiving groove 4115 may be designed to be in a shape of a corner, and the elastic sheet 4114 is designed to be adapted to the elastic sheet receiving groove 4115 having the shape of a corner. With setting of the shape of a corner, the suture 5000 cannot easily slip off the elastic sheet 4114 and is stably fixed.

Referring to FIGS. 21a -21c and FIG. 24, in a further implementation, the adjusting rail 4200 defines a rail cavity 4210 and has a rail outer wall 4220. The handle connecting portion 4120 includes an inserting end 4121 and a connecting shaft 4122. The inserting end 4121 is disposed in the rail cavity 4210 and can move back and forth in the rail cavity 4210. The connecting shaft 4122 is disposed on the rail outer wall 4220 and can move back and forth on the rail outer wall 4220. The inserting end 4121 is fixed to the suture fixation 4110 via the connecting shaft 4122. The inserting end 4121 is disposed in the rail cavity 4210, and movement of the inserting end 4121 is limited to be within the rail cavity 4210 due to the rail outer wall 4220, and thus the inserting end 4121 cannot easily slip off the rail cavity 4210.

As illustrated in FIGS. 25a -25b and FIG. 26, a locking device 100d according to a fifth implementation of the disclosure is illustrated. The locking device 100d differs from the locking device 100 in the first implementation in that the locking device 100d is provided with first teeth 4221 on the rail outer wall 4220 and second teeth 4123 on the connecting shaft 4122. The first teeth 4221 can be adapted to the second teeth 4123 to enable the connecting shaft 4122 to roll on the rail outer wall 4220, thereby driving the bunching device 4100 to move back and forth on the adjusting rail 4200 along the axial direction of the adjusting rail 4200. When the operator rotates the bunching device 4100, the bunching device 4100 moves along the axial direction of the adjusting rail 4210, and a rotational motion of the bunching device 4100 can be converted into a linear motion of the suture 5000, thereby accurately achieving the tightening and loosening of the suture 5000.

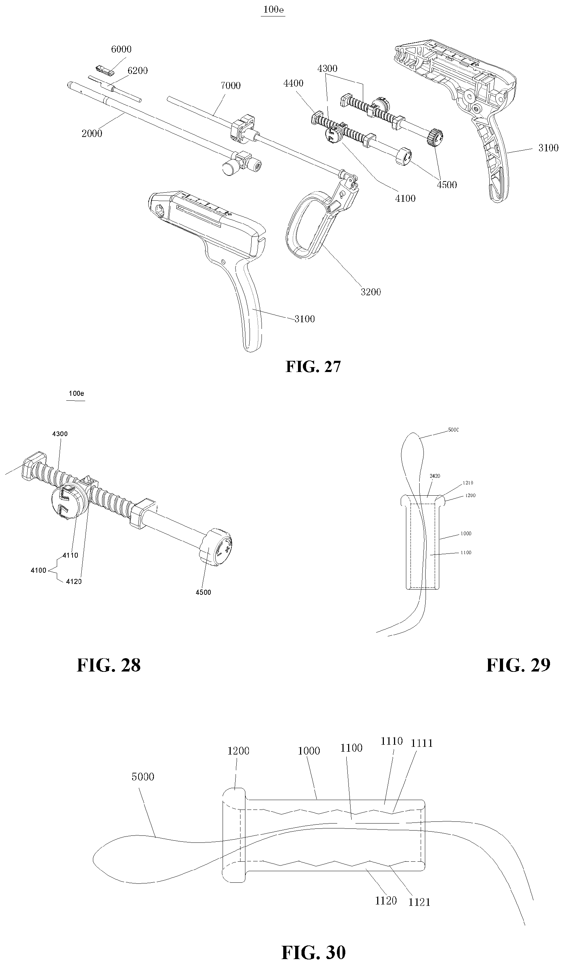

As illustrated in FIGS. 27 -28, a locking device 100e according to a sixth implementation is illustrated. The locking device 100e differs from the locking device 100 in the first implementation in that the adjusting device 4000 further includes a guide screw 4300, a bolt 4400, and an adjusting knob 4500. The guide screw 4300 is fixed in the fixed portion 3100 along the axial direction of the adjusting rail 4200. The bolt 4400 is fixed in the fixed portion 3100, and the guide screw 4300 passes through the bolt 4400 and is adapted to the bolt 4400. A proximal end of the guide screw 4300 passes through the proximal end of the fixed portion 3100 and is coupled to the adjusting knob 4500. The handle connecting portion 4120 is fixed to the guide screw 4300. The adjusting knob 4500 can adjust an axially forward or backward movement of the guide screw 4300. When the operator rotates the adjusting knob 4500, the adjusting knob 4500 drives the guide screw 4300 to rotate in the fixed portion 3100, thereby driving the bunching device 4100 coupled to the guide screw 4300 to move back and forth on the adjusting rail 4200 along the axial direction of the adjusting rail 4200.

Referring back to FIG. 19, in a further implementation, the movable portion 3200 can move with respect to the fixed portion 3100. The lock pin 1000 defines a hollow inner cavity 1100 in the axial direction of the lock pin 1000 (as illustrated in FIG. 29). The hollow inner cavity 1100 is used to receive the suture 5000 and allow the suture 5000 to pass through. The locking assembly 200 further includes a squeezing assembly 6000 and a mandrel 7000 (referring to FIG. 19). The squeezing assembly 6000 is used to hold and press the lock pin 1000 to deform the lock pin 1000 (referring to FIGS. 20a -20b, where FIG. 20a illustrates the lock pin undeformed, and FIG. 20b illustrates the lock pin deformed). When the lock pin 1000 is subjected to a mechanical external force, the lock pin 1000 can be flattened to fix the suture 5000 in the lock pin 1000, and no relative movement occurs between the suture 5000 and the lock pin 1000, thereby locking and fixing the suture 5000. The lock pin 1000 may be of various shapes, for example, may be in the shape of a cylinder, a prism, or the like. As long as the lock pin 1000 defines the hollow inner cavity 1100 for accommodating the suture 5000, the lock pin 1000 may be of any shape. In this implementation, the lock pin 1000 is in the shape of a cylinder to reduce a squeezing resistance.

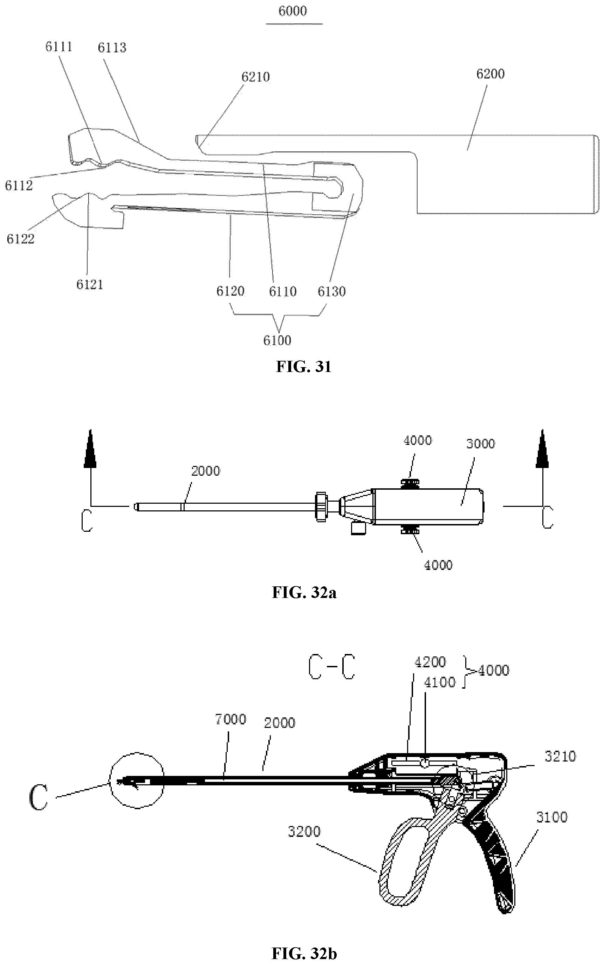

A distal end of the mandrel 7000 is coupled to a proximal end of the squeezing assembly 6000, and a proximal end of the mandrel 7000 is movably coupled to the movable portion 3200. The squeezing assembly 6000 and the mandrel 7000 are both received in the receiving cavity 2100, and the movable portion 3200 can move relative to the fixed portion 3100 to move the mandrel 7000, so as to cause the squeezing assembly 6000 to hold and press the lock pin 1000. It is to be understood that the mandrel 7000 applies a force to the squeezing assembly 6000 during the movement of the mandrel 7000, so that the squeezing assembly 6000 can apply a mechanical external force to hold and press the lock pin 1000. The mandrel 7000 can extend to the fixed portion 3100, and a movable connecting portion 3210 between the movable portion 3200 and the mandrel 7000 is disposed in the fixed portion 3100 (as illustrated in FIGS. 32a - 32b).

As illustrated in FIG. 30, in a further implementation, the hollow inner cavity 1100 of the lock pin 1000 has an upper surface 1110 and a lower surface 1120 opposite the upper surface 1110. A lock pad 1111 protrudes from the upper surface 1110, the lower surface 1120 defines a concave lock hole 1121, and the lock pad 1111 is adapted to the lock hole 1121. It is to be understood that the upper surface and the lower surface are named when the lock pin 1000 is placed horizontally, and when the lock pin 1000 is placed vertically, the upper surface and the lower surface may also be called a left surface and a right surface. When the lock pin 1000 is deformed by an external squeezing force, the convex lock pad 1111 is pressed into the concave lock hole 1121. When the lock pin 1000 is further deformed, the lock pad 1111 and the lock hole 1121 are both deformed until the lock pad 1111 and the lock hole 1121 cannot be separated from each other. At this point, the suture 5000 is firmly fixed in the hollow inner cavity 1100 of the lock pin 1000.

Referring to FIG. 31 and FIGS. 33a -33b, in a further implementation, the squeezing assembly 6000 includes a collet 6100 and a pushing rod 6200. The collet 6100 includes an upper clip 6110, a lower clip 6120, and a clip connecting portion 6130 coupled between a proximal end of the upper clip 6110 and a proximal end of the lower clip 6120. The lock pin 1000 is disposed between the upper clip 6110 and the lower clip 6120. A proximal end of the pushing rod 6200 is coupled to the distal end of the mandrel 7000. A distal end of the pushing rod 7000 is close to the upper clip 6120. The mandrel 7000 drives the pushing rod 6200 to move toward the upper clip 6110, thereby driving the upper clip 6110 to move toward the lower clip 6120 to make the upper clip 6110 and the lower clip 6120 cooperatively hold and press the lock pin 1000.

It is to be understood that, the upper clip 6110 and/or the lower clip 6120 are /is at least partially made from a deformable material and have/has certain elasticity. Therefore, when the upper clip 6110 and/or the lower clip 6120 are/is subjected to an external force, the deformable upper clip 6110 and/or the deformable lower clip 6120 may be driven to be close to each other, so as to hold and press the lock pin 1000 disposed between the upper clip 6110 and the lower clip 6120 to form a shape with a certain curvature. It is to be understood that the upper clip 6110 and the lower clip 6120 are preferably made of stainless steel, nickel titanium alloy, cobalt chromium alloy or the like, and the clip connecting portion 6130 is made of stainless steel, nickel titanium alloy or the like. In this implementation, the whole collet 6100 is made of nickel-titanium alloy.

Still referring to FIG. 31, in a further implementation, the upper clip 6110 has a first surface 6111 that faces the lock pin 1000, and the lower clip 6120 has a second surface 6121 that faces the lock pin 1000. A first engaging portion 6112 is formed on the first surface 6111, a second engaging portion 6122 is formed on the second surface 6121, and the first engaging portion 6112 can be adapted to the second engaging portion 6122 to enable the upper clip 6110 and the lower clip 6120 to be in a close state. It is to be understood that the first engaging portion 6112 and the second engaging portion 6122 each may have a curvature shape or a sawtooth shape and can cooperate with each other.

When the lock pin 1000 is provided with a circular cone 1200 at the distal end thereof (see FIG. 30), the diameter of the circular cone 1200 is larger than that of the proximal end of the lock pin 1000. In order to cooperate with the lock pin 1000 with the circular cone 1200, in a further implementation, axial lengths of the upper clip 6110 and the lower clip 6120 are different from each other. Preferably, a difference between the axial length of the upper clip 6110 and the axial length of the lower clip 6120 equals a preset length. The preset length is at least equal to the thickness of the circular cone 1200. Thus, the circular cone 1200 of the lock pin 1000 non-pressed and the collet 6100 can be both placed at the distal end of the outer tube 2000 without affecting a size of the outer tube 2000. The thickness of the circular cone 1200 refers to the length of the circular cone 1200 along the axial direction of the lock pin 1000.

In a further implementation, the distal end of the pushing rod 6200 has a first inclined surface 6210 (referring to FIG. 31), and the first inclined surface 6210 is inclined downwardly along a direction from the distal end to the proximal end. The upper clip 6110 has a second inclined surface 6113 that faces away from the lock pin 1000, and the second inclined surface 6113 is inclined downwardly along a direction from the distal end to the proximal end to ensure that there is a relatively large contact area between the collet 6100 and the pushing rod 6200. Preferably, a slope of the first inclined surface 6210 is smaller than that of the second inclined surface 6113. The slope of the first inclined surface 6210 refers to an included angle defined between the first inclined surface 6210 and a direction from the distal end of the pushing rod 6200 to the proximal end of the pushing rod 6200, and the slope of the second inclined surface 6113 refers to an included angle defined between the second inclined surface 6113 and a direction from the distal end of the pushing rod 6200 to the proximal end of the pushing rod 6200, and thus during that the pushing rod 6200 is moved toward the distal end, the first inclined surface 6210 at the distal end of the pushing rod 6200 can continuously squeeze the second inclined surface 6113 of the upper clip 6110, so that the upper clip 6110 gradually gets close to the lower clip 6120, thereby continuously deforming the lock pin 1000 until the lock pin 1000 can no longer be deformed. At this point, the lock pin 1000 and the suture 5000 are firmly fixed together. The pushing rod 6200 is made of stainless steel, nickel-titanium alloy, or cobalt-chromium alloy, and is preferably made of stainless steel.

It is to be understood that the distal end of the pushing rod 6210 and the proximal end of the pushing rod 6210 form an L-shaped structure, that is, a radial thickness of the distal end of the pushing rod 6210 is smaller than that of the proximal end of the pushing rod 6210, and the distal end of the pushing rod 6210 is at one side of the whole pushing rod 6210, such that the distal end of the pushing rod 6210 and the proximal end of the pushing rod 6210 form the L-shaped structure. When the pushing rod 6200 is advanced to hold and press the collet 6100 downwardly, the L-shaped pushing rod 6200 enables the collet 6100 in the pressed state to be disposed in the L-shaped structure, such that a difference between a thickness of the whole structure formed by the pushing rod 6200 when the collet 6100 is pressed and a thickness of the collet 6100 non-pressed is relatively small, thereby not affecting the size of the outer tube 2000.

It is to be understood that the pushing rod 6210 may be a hollow tube or have a solid rod-like structure.

Referring to FIGS. 32a-32b, FIG. 33a, and FIG. 33b, in a further implementation, the distal end of the outer tube 2000 defines a suture inlet 2200 along the radial direction of the outer tube 2000. A diameter of the suture inlet 2200 is at least equal to the diameter of a portion of the lock pin 1000, where the portion of the lock pin 1000 has a maximum outer diameter, and thus the lock pin 1000 pressed can slip off the distal end of the outer tube 2000. The outer tube 2000 further defines a suture outlet 2300. The suture inlet 2200 and the suture outlet 2300 communicate with the hollow inner cavity 1100 of the lock pin 1000, respectively, so that the proximal end of the suture 5000 can sequentially pass through the suture inlet 2200, the proximal end of the lock pin 1000, and the suture outlet 2300. It is to be understood that, in other implementations, the suture outlet 2300 may be in any position of the outer tube 2000 or in the handle 3000, as long as the suture outlet 2300 communicates with the hollow inner cavity 1100 of the lock pin 1000, i.e., the proximal end of the suture 5000 can pass through the suture outlet 2300.

In a further implementation, the outer tube 2000 is provided with a holding portion 2400 at the distal end thereof, and the diameter of the holding portion 2400 gradually decreases from the proximal end to the distal end. The holding portion 2400 is used to prevent the lock pin 1000 non-pressed from slipping off the distal end of the outer tube 2000. The diameter of the holding portion 2400 gradually reduces from the proximal end to the distal end, thereby facilitating driving the distal end of the outer tube 2000 to enter the patient's body and smoothly move in the body.

As illustrated in FIGS. 34a and 34b, in a further implementation, the holding portion 2400 has a holding portion end surface 2410 at the distal end thereof. The holding portion 2400 defines an opening 2420 in the axial direction of the holding portion 2400, and the opening 2420 extends through the holding portion end surface 2410 and communicates with the receiving cavity 2100. The opening 2420 is the suture inlet 2200.

In a further implementation, a protrusion 2430 is formed at the opening 2420 in a radial direction of the opening 2420 (see FIG. 33a). A preset distance exists between the protrusion 2430 and the holding portion end surface 2410. The lock pin 1000 is partially disposed in the opening 2420, so that the protrusion 2430 can fix the lock pin 1000.

As illustrated in FIG. 34a, FIG. 34b, FIG. 33a, FIG. 33b, and FIG. 29, in a further implementation, the lock pin 1000 is provided with the circular cone 1200 at the distal end thereof. The diameter of the circular cone 1200 is larger than that of the proximal end of the lock pin 1000, and the circular cone 1200 is disposed in the opening 2420. The protrusion 2430 fixes the lock pin 1000, thereby preventing the lock pin 1000 from slipping off the distal end of the outer tube 2000. With the circular cone 1200, the transition between the hollow inner cavity 1100 of the lock pin 1000 and the distal end of the lock pin 1000 is enabled to be smooth to the greatest extent, thereby preventing a connection portion between the hollow inner cavity 1100 and the distal end of the lock pin 1000 from cutting off the suture 5000 or scratching internal tissues of the patient's body. It is to be understood that the preset distance is at least equal to the thickness of the circular cone 1200, so that the circular cone 1200 is arranged between the protrusion 2430 and the holding portion end surface 2410, thereby facilitating stability of the lock pin 1000.

As illustrated in FIG. 29, in a further implementation, the circular cone 1200 has an inner sidewall 1210 that is an arc-shaped surface, such that the suture 5000 can smoothly pass through the lock pin 1000, thereby preventing the lock pin 1000 from cutting off the suture 5000 or damaging human tissues.

In a further implementation, the outer tube 2000 and the mandrel 7000 may both be manufactured by processing a metal material such as stainless steel, nickel titanium, pure titanium or the like, or can be manufactured by processing a polymer material such as acrylonitrile butadiene styrene (ABS), polystyrene (PS), polyether ether ketone (PEEK), or the like. The outer tube 2000 and the mandrel 7000 may be made from the same material or different materials. The outer tube 2000 and the mandrel 7000 may be preferably made of stainless steel.

In a further implementation, the lock pin 1000 is made of stainless steel, pure titanium, nickel titanium, cobalt chromium alloy, or the like, and preferably made of pure titanium or stainless steel.

When using the locking device 100, the operator first enables the proximal end of the suture 5000 to sequentially pass through the suture inlet 2200 on the distal end of the outer tube 2000, the opening on the distal end of the lock pin 1000, and the suture outlet 2300 on the distal end of the outer tube 2000, and then enables the suture 5000 to be fixed to the suture fixation 4110 of the bunching device 4100. The length of the suture 5000 is then adjusted by the adjusting device 4000 according to need. After adjustment, the movable portion 3200 of the handle 3000 is driven to move toward the fixed portion 3100, so as to drive the mandrel 7000 to move toward the distal end with respect to the outer tube 2000, thereby causing the pushing rod 6200 to hold and press the collet 6100. The upper clip 6110 and the lower clip 6120 of the collet 6100 hold and press the lock pin 1000 to deform the lock pin 1000 (referring to FIG. 33a and FIG. 33b, where FIG. 33a illustrates the lock pin 1000 non-pressed, and FIG. 33b illustrates the lock pin 1000 pressed), and then the suture 5000 received in the lock pin 1000 is fixed to the lock pin 1000.

The following will illustrate an operation process of the locking device 100 in the disclosure by taking the mitral valve that has lesions as an example.

The mitral valve is a one-way "valve" between a left atrium (LA for short) and a left ventricle (LV for short) and can ensure blood flow from the left atrium to the left ventricle. The mitral valve in a health state has chordae tendineae. The mitral valve includes two leaflets, i.e., an anterior leaflet and a posterior leaflet. When the left ventricle is in a diastolic state, the anterior leaflet and the posterior leaflet are in an open state, and the blood flows from the left atrium to the left ventricle. When the left ventricle is in a contracted state, the chordae tendineae are stretched to prevent the leaflets from being washed, by the blood flow, to the left atrium, and the anterior leaflet and the posterior leaflet are in a good close state to ensure blood flow from the left ventricle to an aorta through an aortic valve (AV for short). Different from the mitral valve in the health state, under the condition that the mitral valve has lesions, the mitral valve cannot return to the close state when the left ventricle is in the contracted state, and the momentum of the blood flow can further cause the leaflet to fall into the left atrium, thereby resulting in blood regurgitation. A usage process of repairing the leaflets of the mitral valve by the locking device 100e provided by the sixth implementation of the disclosure is as follows. The locking device 100e in the sixth implementation can include features of the locking feedback element 8000 in any of the above mentioned implementations.

At the first step, multiple sutures 5000 with elastic pads are first implanted into both the anterior leaflet and the posterior leaflet of the mitral valve (see FIG. 35). A point contact between the suture 5000 and the leaflet is changed to a surface contact between the elastic pad and the leaflet, thereby effectively reducing a risk of tearing the leaflet.

At the second step, outside the patient's body, the multiple sutures 5000 in both the leaflets are all inserted into the lock pin 1000 of the locking device 100e, and the proximal ends of the sutures 5000 are enabled to pass through the suture outlet 2300 on the distal body of the outer tube 2000 (see FIG. 36). The sutures in the anterior leaflet and posterior leaflet are distinguished, and the proximal ends of the two sets of sutures 5000 are respectively wound on the spools of the bunching devices 4100 several times, and then the proximal ends of the sutures 5000 are fixed to the elastic sheets 4114 to maintain the relative position between the sutures 5000 and the lock pin 1000 (see FIG. 37).

At the third step, the distal end of the locking device 100e is pushed into the heart through the apex of the heart and is moved to be close to the leaflet of the mitral valve, meanwhile, the sutures 5000 are pulled, until the distal end of the locking device 100e reaches a predetermined position that is below the leaflet (see FIG. 37).

At the forth step, the two adjusting knobs 4500 are rotated respectively to drive the two guide screws 4300 to rotate, thereby driving each bunching device 4100 connected to one guide screw 4300 to move back and forth on the adjusting rail 4200 along the axis direction of the adjusting rail 4200, so as to adjust the tightness of the two sets of sutures 5000 coupled to the bunching devices 4100 respectively, and determine, by ultrasound, a state in which the mitral regurgitation is slightest. When the state reaches, the rotation of the adjusting knobs 4500 are stopped to maintain the tightness of the two sets of sutures 5000, that is, to maintain a relative distance between the anterior leaflet and the posterior leaflet.

At the fifth step, as illustrated in FIG. 32a, FIG. 32b, FIG. 33a, FIG. 33b, and FIG. 37, the fixed portion 3100 of the handle 3000 remains stationary, and the movable portion 3200 is driven to move toward the fixed portion 3100 until the movable portion 3200 triggers the locking feedback element 8000 to give an audible feedback. At this point, the mandrel 7000 and the pushing rod 6200 move toward the distal end with respect to the outer tube 2000. The distal end of the pushing rod 6200 continuously squeezes the collet 6100, so that the upper clip 6110 and the lower clip 6120 of the collet 6100 get close to each other to hold and press the lock pin 1000 between the upper clip 6110 and the lower clip 6120, until the lock pin 1000 is deformed and the sutures 5000 in the lock pin 1000 are fixed in the lock pin 1000. The movable portion 3200 is loosened, the elastic collet 6100 opens automatically. The deformed lock pin 1000 slips off the suture inlet 2200 on the distal end of the outer tube 2000 of the locking device 100e.

At the sixth step, the distal end of the locking device 100e is pulled out of the patient's body, the lock pin 1000 stays in the patient's body, and the proximal end of the sutures 5000 is fixed to a ventricular wall. At this point, with the lock pin 1000, the two sets of sutures 5000 in the anterior leaflet and the posterior leaflet are fixed together (see FIG. 38), and the anterior leaflet and the posterior leaflet of the mitral valve are pulled toward each other, edge-to-edge repair is completed, thereby forming a dual-bore structure.

It is to be understood that operation processes of other implementations are similar to that of the sixth implementation, which will not be repeated herein.

Referring to FIG. 39, a heart valve repair system 10 is provided. The heart valve repair system 10 includes the locking device provided in any of the above implementations and at least one suture 5000. The suture 5000 is configured to be implanted to a valve leaflet of a heart valve.

In a further implementation, the heart valve repair system 10 further includes a suture implantation device 400 which is configured to implant the suture 5000 to the valve leaflet of the heart valve. The locking device 100 is configured to fix the suture 5000 on an apex of the heart, a papillary muscle, or a ventricular wall, where the suture 5000 is used as an artificial chordae to form a "chordal repair".

In other implementations, the suture implantation device 400 is configured to implant the suture 5000 to different valve leaflets of the heart valve. The locking device 100 is configured to fix multiple sutures 5000 together, so as to pull the leaflets toward each other, thereby forming an "edge-to-edge repair".

The heart valve repair system 10 can lock the suture 5000 by the locking device 100 and can also judge whether the suture 5000 is locked according to the locking feedback element 8000 in the locking device 100 to ensure a locking force on the suture 500, so that the conditions that the suture is not locked and that the suture is excessively locked to be damaged and broken and fail may be prevented, a knot locking effect is ensured, and the success rate of the operation is increased.

The implementations of the disclosure are described in detail above, and specific examples are used to explain the principles and implementations of the disclosure. The illustration of the above implementations is only used to help in understanding the method and the core idea of the disclosure. Also, according to the ideas of the disclosure, those of ordinary skill in the art can make changes for specific implementations and application scopes. In summary, the content of this specification should not be construed as limiting the disclosure.

## Claims

1. A locking device with a locking feedback function, comprising:
a locking assembly configured to lock a target object;
a handle coupled with the locking assembly, wherein the handle comprises a movable portion and a fixed portion, and the movable portion is configured to move relative to the fixed portion to drive the locking assembly to lock the target object; and
a locking feedback element movably disposed in the fixed portion and configured to cooperate with the movable portion of the handle to feed back whether the target object is locked.

2. The locking device of claim 1, wherein the locking feedback element is operable to be exposed outside the fixed portion or impact with the fixed portion to make a sound, to feed back whether the target object is locked.

3. The locking device of claim 1, wherein
the locking feedback element comprises a feedback sliding block, a supporting element, and an elastic element coupled between the feedback sliding block and the supporting element;
the feedback sliding block is operable to move toward a proximal end of the fixed portion under an action of the movable portion, the supporting element is capable of moving with the feedback sliding block;
the fixed portion defines a first notch thereon; and
when the supporting element moves to and fall in the first notch, the locking feedback element is exposed outside the fixed portion or impacts with the fixed portion to make a sound, to feed back that the target object is locked.

4. The locking device of claim 3, wherein
the fixed portion defines a first limiting groove and a second limiting groove therein;
the feedback sliding block is operable to move in the first limiting groove toward the proximal end of the fixed portion under the action of the movable portion;
the supporting element is operable to move in the second limiting groove toward the same direction as the feedback sliding block;
the first notch is in communication with the second limiting groove, the first notch is formed adjacent to the proximal end of the fixed portion, and extends through an outer surface of a bottom end of the fixed portion; and
when being moved to face the first notch, the supporting element falls in the first notch under the action of the elastic element.

5. The locking device of claim 4, wherein
the locking feedback element further comprises an impact element, the elastic element is elastically abutted against the impact element, and the supporting element is disposed on a side the impact element away from the elastic element; and
when the supporting element is moved to face the first notch, the elastic element drives the impact element to impact with the fixed portion to make a sound to feed back that the target object has been locked, and the supporting element falls in the first notch.

6. The locking device of claim 5, wherein a cross sectional area of the impact element is larger than a cross sectional area of the first notch, such that the impact element is abutted against a part above the first notch when the supporting element falls in the first notch.

7. The locking device of claim 6, wherein
the second limiting groove comprises a first supporting surface, the supporting element is operable to slide on the first supporting surface, and the first notch extends through the first supporting surface; and
when the supporting element falls in the first notch, the impact element is positioned on the first supporting surface around the first notch.

8. The locking device of claim 5, wherein
the locking feedback element further comprises at least one inverted buckle, the inverted buckle comprises a first connecting portion and a clamping portion, and the clamping portion is coupled to the feedback sliding block via the first connecting portion and disposed below the feedback sliding block;
an orthographic projection of the impact element on the feedback sliding block is at least partially overlapped with an orthographic projection of the clamping portion on the feedback sliding block; and
when the supporting element falls in the first notch, the impact element is abutted against the clamping portion.

9. The locking device of claim 8, wherein
there are two inverted buckles arranged on two opposite sides of the impact element; and
when the supporting element falls in the first notch, the impact element is abutted against the clamping portions of the two inverted buckles.

10. The locking device of any one of claims 4-9, wherein
the locking feedback element further comprises a feedback button, the feedback button comprises a button portion and a second connecting portion, the button portion is coupled to the impact element via the second connecting portion, and the button portion is exposed outside the outer surface of the bottom end of the fixed portion.

11. The locking device of claim 10, wherein
the fixed portion further comprises a second notch, and the second notch extends along the second limiting groove, and extends through the second limiting groove and the outer surface of the bottom end of the fixed portion; and
the second connecting portion extends from the second notch, and when the feedback sliding block slides in the first limiting groove, the second connecting portion moves in the second notch to drive the button portion exposed outside the bottom end of the fixed portion to move.

12. The locking device of claim 10, wherein, when the supporting element is abutted against the first supporting surface of the second limiting groove, a distance between the button portion and the outer surface, away from the second limiting groove, of the fixed portion is less than a preset distance.

13. The locking device of any one of claims 4-9, wherein the fixed portion further defines a third notch therein, the third notch extends in a direction which is consistent with a moving direction of the feedback sliding block, and the movable portion is configured to drive the feedback sliding block to move via the third notch.

14. The locking device of claim 13, wherein a lug boss is arranged at a position, corresponding to the third notch, of the movable portion, and when the movable portion moves to the fixed portion, the lug boss is abutted against the feedback sliding block, and drives the feedback sliding block to move in the first limiting groove.

15. The locking device of claim 14, wherein the lug boss comprises a first junction portion and a second junction portion, and the first junction portion and the second junction portion are operable to extend and retract relative to each other to adjust an axial length of the lug boss.

16. The locking device of claim 15, wherein the first junction portion and the second junction portion are operable to extend and retract relative to each other in a threaded extension and retraction manner or a buckle extension and retraction manner.

17. The locking device of claim 16, wherein
the first junction portion and the second junction portion are operable to extend and retract relative to each other in the threaded extension and retraction manner, the first junction portion is one of a screw element and a screw cap adapted to the screw element, and the second junction portion is the other of the screw element and the screw cap.

18. The locking device of claim 13, wherein
when the supporting element is located in the second limiting groove, the feedback sliding block extends outside an outer surface of the fixed portion through the third notch.

19. The locking device of claim 18, wherein a length-adjustable telescopic portion is further disposed at an end, extending outside the outer surface of the fixed portion from the third notch, of the feedback sliding block.

20. The locking device of any of claims 1-9, wherein
the target object comprises a lock pin and a suture, the lock pin is configured to accommodate or fix the suture, and the locking assembly is configured to lock the suture in the lock pin; and
the locking assembly comprises:
an outer tube defines a receiving cavity therein, the lock pin being disposed at a distal end of the receiving cavity, and the fixed portion being coupled to a proximal end of the outer tube; and
an adjusting device disposed on the fixed portion, the adjusting device being coupled to a proximal end of the suture and configured to adjust tightening or loosening of the suture.

21. The locking device of claim 20, wherein
the adjusting device comprises a bunching device and an adjusting rail, the adjusting rail being disposed on the fixed portion, and the bunching device is coupled to the fixed portion via the adjusting rail; and
the bunching device comprises a suture fixation and a handle connecting portion coupled to the suture fixation, wherein the suture fixation is detachably coupled with the suture, the handle connecting portion is disposed in the adjusting rail, and the bunching device is able to move back and forth, via the handle connecting portion, on the adjusting rail along an axial direction of the adjusting rail to adjust tightening or loosening of the suture.

22. The locking device of claim 21, wherein the adjusting rail comprises a rail cavity and a rail outer wall, the handle connecting portion comprises an inserting end and a connecting shaft, the inserting end is disposed in the rail cavity and able to move back and forth in the rail cavity, the connecting shaft is disposed on the rail outer wall and able to move back and forth on the rail outer wall, and the inserting end is coupled to the suture fixation via the connecting shaft.

23. The locking device of claim 21, wherein
the adjusting device further comprises a guide screw, a bolt, and an adjusting knob, wherein the guide screw is fixed in the fixed portion along the axial direction of the adjusting rail, the bolt is fixed in the fixed portion, and the guide screw passes through the bolt and is adapted to the bolt; and
a proximal end of the guide screw passes through the proximal end of the fixed portion and is coupled to the adjusting knob, the handle connecting portion is fixed on the guide screw, and the adjusting knob is able to adjust an axially forward or backward movement of the guide screw.

24. A heart valve repair system, comprising the locking device of any of claims 1-23 and at least one suture, the suture being configured to be implanted to a valve leaflet of a heart valve.

25. The heart valve repair system of claim 24, further comprising a suture implantation device, wherein the suture implantation device is configured to implant the suture to different valve leaflets of the heart valve, and the locking device is configured to fix multiple sutures together.

26. The heart valve repair system of claim 24, further comprising a suture implantation device, wherein the suture implantation device is configured to implant the suture to the valve leaflet of the heart valve, and the locking device is configured to fix the suture on an apex of the heart, a papillary muscle, or a ventricular wall.
